(19) Europäisches Patentamt
European Patent Office
Office européen des brevets

(11) Numéro de publication: **0 033 432**
**B1**

(12) # FASCICULE DE BREVET EUROPEEN

(45) Date de publication du fascicule du brevet: 28.09.83

(21) Numéro de dépôt: 80401898.4

(22) Date de dépôt: 31.12.80

(51) Int. Cl.³: C 07 D 233/60, C 07 D 401/12, A 61 K 31/415

(54) **Composition thérapeutique à base d'imidazoles destinée notamment au traitement des thromboses, nouveaux imidazoles et leur procédé de préparation.**

(30) Priorité: 31.01.80 FR 8002057

(43) Date de publication de la demande: 12.08.81 Bulletin 81/32

(45) Mention de la délivrance du brevet: 28.09.83 Bulletin 83/39

(84) Etats contractants désignés: AT BE CH DE FR GB IT LI LU NL SE

(56) Documents cités:
E. HAYEK et al.: "MONATSHEFTE FÜR CHEMIE", vol. 108, 1977, Springer Verlag VIENNE (AU) M. PAILER et al.: "Synthese der rac. 1-(3-Hydroxy-1-(E)-octenyl)-2-imidazol-heptansäure" pages 653-664
PROSTAGLANDINS, vol. 13, no. 4, avril 1977 S. MONCADA et al.: "Imidazole: a selective inhibitor of thromboxane synthetase" pages 611-618

Le dossier contient des informations techniques présentées postérieurement au dépôt de la demande et ne figurant pas dans le présent fascicule.

(73) Titulaire: LABORATOIRES CHAUVIN-BLACHE Société dite
Rue de la Galéra
F-34000 Montpellier (FR)

(72) Inventeur: Bonne, Claude
12 bis Avenue Aristide Briand
F-94360 Bry sur Marne (FR)
Inventeur: Coquelet, Claude
24 La Gaillarderie
F-78590 Noisy-le-Roi (FR)
Inventeur: Sincholle, Daniel
343 Avenue de la Trémoulette
F-34980 St Clement (FR)

(74) Mandataire: Bressand, Georges et al,
c/o CABINET LAVOIX 2 Place d'Estienne d'Orves
F-75441 Paris Cedex 09 (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 033 432 B1

Composition thérapeutique à base d'imidazoles destinée notamment au traitement des thromboses, nouveaux imidazoles et leur procédé de préparation

La présente invention concerne une nouvelle composition thérapeutique ayant une activité anti-agrégante plaquettaire et anti-thrombotique.

On sait, depuis 1976, que la paroi vasculaire contribue de façon primordiale au maintien des plaquettes dans un état inactivé, en produisant la prostacycline, $PGI_2$.

La $PGI_2$ est synthétisée à partir de l'acide arachidonique dans les cellules de la paroi vasculaire. Ce même précurseur, dans les plaquettes, est converti en prostaglandines $E_2$, $D_2$ et $F_2$ et en thromboxane $A_2$ qui est un métabolite extrêmement agrégant. Ces deux voies métaboliques antagonistes au plan fonctionnel, passent par les mêmes produits intermédiaires, les endopéroxydes $PGG_2$ et $PGH_2$.

Cette double synthèse des prostaglandines à partir de l'acide arachidonique (AA) dans les cellules pariétales vasculaires et dans les plaquettes peut être illustrée par le schéma représenté sur la fig. 1.

Le métabolisme de l'acide arachidonique en prostaglandines et thromboxanes est inhibé par l'aspirine dès la première étape (cyclo-oxygénase). Ce produit empêche donc l'agrégation plaquettaire par la voie des thromboxanes, mais inversement, en réduisant la synthèse de la prostacycline par la paroi vasculaire, il augmente las sensibilité des plaquettes aux agents thrombogènes.

Il est apparu préférable de bloquer la formation des thromboxanes dans les plaquettes plus en aval dans la cascade métabolique et l'on a cherché à inhiber la thromboxane synthétase.

A cet effet, on a déjà proposé différents produits et notamment l'imidazole (Moncada et col. Prostaglandines *13* 611—618, 1977).

Tai et Yuan (Biochem. Biophys. Res. Comm. *80* 236—242, 1978) ont étudié divers dérivés de l'imidazole et ont montré que cette activité inhibitrice était importante pour les dérivés substitués en position 1 par une chaîne alkyle ou aryle.

La présente invention est basée sur la découverte que les composés de formule générale

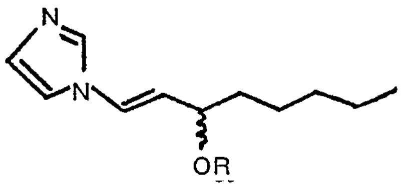

(I)

dans laquelle R représente un atome d'hydrogène ou un rest d'éther ou d'ester, ainsi que leurs sels d'addition avec des acides pharmaceutiquement acceptables, possèdent une forte activité inhibitrice spécifique sur la thromboxane synthétase tout en permettant d'augmenter la synthèse de prostacycline $PGI_2$.

Les études pharmacologiques ont confirmé que cette activité inhibitrice se traduisait par une activité anti-agrégante plaquettaire et une activité anti-thrombotique.

La présente invention a en conséquence pour objet une composition thérapeutique ayant une activité inhibitrice sur la thromboxane synthétase, une activité anti-agrégante plaquettaire et une activité anti-thrombotique caractérisée en ce qu'elle contient à titre de principe actif un composé de formule I ou l'un de ses sels d'addition avec des acides pharmaceutiquement acceptables.

Les sels d'addition peuvent notamment être ceux formés avec les acides halohydrique, sulfurique, nitrique, phosphorique, formique, acétique, fumarique, oxalique, malique, méthane sulfonique, lactique, succinique, tartrique, pamoïque et les sels métalliques acides tels que l'orthophosphate disodique et le sulfate monopotassique.

Les esters sont notamment les composés de formule

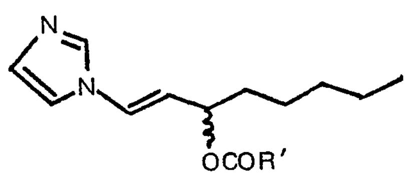

(II)

et les éthers les composés de formule

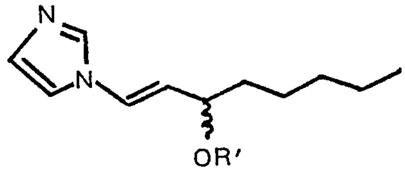

(III)

formules dans lesquelles R′ représente un radical alkyle en $C_1$ à $C_8$, un radical aryle, aryl(alkyle en $C_1$—$C_5$) ou (alkyl en $C_1$—$C_5$) aryle, le terme aryle désignant un radical hydrocarboné aromatique ayant 6 à 10 atomes de carbone, tel qu'un radical phényle, ou un radical hétéroaromatique contenant un ou deux hétéroatomes choisis parmi l'azote, l'oxygène et le soufre et ayant de 5 à 11 chaînons, tel qu'un radical pyridyle ou thiényle.

Les composés de formule I peuvent être utilisés sous forme de mélange racémique ou d'isomères optiques.

Le (E)-(hydroxy-3 octèn-1-yl)-1-imidazole (racémique) a été décrit par Pailer et Gutwillinger (Monatshefte für Chemie *108*, 653—664, 1977) sans qu'aucune application thérapeutique n'ait été envisagée. Le chlorhydrate de ce racémique (composé A) est une poudre blanche, F = 98—100°C.

Les éthers et esters de l'(hydroxy-3 octèn-1-yl)-1 imidazole sont des composés nouveaux.

Ces composés peuvent être préparés selon des procédés connus, notamment par réaction sur l'alcool correspondant (composé de formule I dans laquelle R = H) d'un dérivé halogéné RX dans lequel R désigne un reste d'éther ou d'ester. Ainsi, pour préparer les esters de formule II, on fait réagir sur l'alcool correspondant un halogénure de formule R′COX dans laquelle R′ a la signification donnée ci-dessus et pour préparer les éthers de formule III on fait réagir sur l'alcool correspondant un dérivé halogéné de formule R′X dans laquelle R′ a la signification donnée ci-dessus.

La réaction peut être effectuée dans un solvant anhydre peu polaire comme le benzène ou le tétrahydrofuranne, en présence d'un agent alcalin, tel que la triéthylamine, le carbonate de potassium anhydre ou l'hydrure de sodium en dispersion dans l'huile.

Le rapport molaire entre l'alcool, le dérivé halogéné et l'agent alcalin est de préférence 1:1,3:1,4 dans le mélange de réaction.

La réaction a lieu en général par chauffage et le temps de chauffage nécessaire pour rendre la réaction complète est d'environ 4 à 16 heures, après l'introduction du dérivé halogéné dans le mélange réactionnel.

Les sels sont obtenus de manière classique par action sur les composés de formule I en solution dans un solvant tel qu'un alcool, une cétone ou un éther aliphatique de l'acide correspondant.

Les exemples suivants illustrent la préparation des composés nouveaux.

## Exemple 1

Dichlorhydrate de l'ester nicotinique du (E)-(hydroxy-3-octèn-1-yl)-1-imidazole

On ajoute 0,03 mole de (E)-(hydroxy-3-octèn-1-yl)-1-imidazole en solution dans 50 ml de benzène à un mélange de 0,04 mole de chlorure de nicotinoyle et de 0,05 mole de triéthylamine dans 100 ml de benzène. On porte à ébullition le mélange réactionnel pendant 12 heures. Après refroidissement on essore le chlorhydrate de triéthylamine et on lave la solution benzénique avec de l'eau. On extrait la phase organique par de l'acide chlorhydrique dilué et on lave la solution aqueuse avec de l'éther éthylique. La solution éthérée est séchée et le solvant évaporé sous vide.

L'ester nicotinique se présentant sous forme huileuse est obtenu avec un rendement de 67%.

Le dichlorhydrate de l'ester précédent est obtenu par passage d'un courant d'acide chlorhydrique gazeux dans une solution éthérée de l'ester. Après avoir essoré le dichlorhydrate brut précipité, ce dernier est recristallisé dans un mélange méthanol-éther éthylique.

Le dichlorhydrate de l'ester nicotinique du (E)-(hydroxy-3-octèn-1-yl)-1-imidazole pur se présente sous forme de cristaux blancs fondant à 145—148°C.

Propriétés spectroscopiques de la base libre

I.R. (film) : $\sqrt{}$ = 1720 $cm^{-1}$ bande C = 0 intense.

R.M.N. ($CDCl_3$) : $\delta$ = 0,42 à 2,08 ppm 11 H (massif); $\delta$ = 5,48 ppm 1 H (quartet); $\delta$ = 5,68 ppm 1 H (quartet, proton vinylique); $\delta$ = 6,82 à 7,18 ppm 3 H (massif, 2 protons imidazoliques, 1 proton vinylique); $\delta$ = 7,28, 8,18, 8,65, 9,13 ppm 4 H (protons nicotiniques).

## Exemple 2

Fumarate de l'éther pyridyl-2 méthylique du (E)-(hydroxy-3 octèn-1-yl)-1-imidazole

On ajoute à température ambiante 0,042 mole d'hydrure de sodium en dispersion dans l'huile (20%) à une solution de 0,030 mole de (E)-(hydroxy-3-octèn-1-yl)-1-imidazole dans 70 ml de tétrahydrofuranne anhydre. On chauffe l'ensemble pendant deux heures à 60°C et on ajoute 0,036 mole de chlorométhyl-2 pyridine en solution dans 30 ml de tétrahydrofuranne anhydre. On maintient le mélange réactionnel à 60°C pendant trois heures après la fin de l'addition. Après évaporation sous vide du solvant, le résidu est trituré dans l'éther éthylique, la solution éthérée lavée avec de l'eau, puis séchée et évaporée sous vide. L'éther brut est obtenu avec un rendement de 85% (huile).

Il peut être purifié par chromatographie sur colonne de silice en utilisant l'acétate d'éthyle comme éluant.

Le fumarate de l'éther précédent est préparé par chauffage à 50°C pendant quinze minutes de quantités équimolaires d'acide fumarique et d'éther brut en solution méthanolique.

Après évaporation sous vide du méthanol, le résidu est trituré dans l'éther éthylique. Le précipité

de fumarate de l'éther pyridyl-2 méthylique du (E)-(hydroxy-3-octèn-1-yl)-1-imidazole est essoré, puis recristallisé dans l'acétate d'éthyle.

Le sel pur obtenu avec un rendement de 62% se présente sous forme de cristaux blancs fondant à 82—85°C.

Propriétés spectroscopiques de la base libre

R.M.N. ($CDCl_3$) $\delta$ = 0,67 à 2,00 ppm 11 H (massif); $\delta$ = 3,98 ppm 1 H (quartet); $\delta$ = 4,65 ppm 2 H (quartet, méthylène en $\alpha$ du noyau pyridinique); $\delta$ = 5,73 ppm 1 H (quartet proton vinylique) $\delta$ = 6,77 à 7,93 ppm 7 H (massif, protons imidazoliques, 3 protons pyridiniques, 1 proton vinylique); $\delta$ = 8,58 ppm (1 proton pyridinique).

Les caractéristiques des composés de formule I préparés aux exemples 1 et 2 et celles d'autres composés préparés de manière analogue sont rassemblées dans le tableau I ci-après.

TABLEAU I

| Composé | R | F (°C) | Rdt (%) | Sel formé | F (°C) | Solvant cristallisation |
|---|---|---|---|---|---|---|
| B | 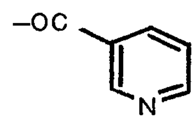 | huile | 77 | di-chlorhydrate | 145–8 | Méthanol-Ether |
| C | 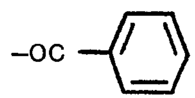 | huile | 52 | Oxalate | 110–13 | Acétate d'éthyle-Ether |
| D | $-OC-CH_3$ | huile | 92 | Oxalate | 76–8 | Méthanol-Ether |
| E | $-OC-(CH_2)_4-CH_3$ | huile | 50 | Oxalate | 61–2 | Méthanol-Ether |
| F | 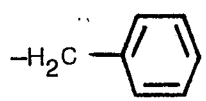 | 66 | 50 | – | – | – |
| G | 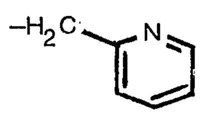 | huile | 67 | Fumarate | 82–5 | Acétate d'éthyle |

On donnera ci-après des résultats de l'étude pharmacologique et toxicologique effectuée avec les composés de formule I

1 — Pharmacologie biochimique

1.1. Inhibition de la thromboxane synthétase plaquettaire

Du plasma riche en plaquettes (PRP) est préparé par centrifugation à 150 g × 10 min. de sang veineux. Les plaquettes sont sédimentées par centrifugation à 2000 g × 20 min. et lavées dans un tampon tris HCl pH 7.5 (15 mM) contenant: EDTA (1,5 mM), NaCl (150 mM) et KCl (5 mM). Le culot plaquettaire est ensuite mis en suspension dans un volume de tris-HCl (50 mM) pH 7.5 correspondant à la moitié du volume de PRP initial. Des parties aliquotes de suspension plaquettaire (0,5 ml) sont incubées avec 1 $\mu$g d'acide $^{14}$Carachidonique (AS = 50 mCi/mmole) sous forme de sel de Na pendant 10 min. à 37°C en présence ou en absence de produit à tester. A la fin de la réaction, l'incubat est porté à 0—4°C, acidifié et salé par addition d'acide citrique et de NaCl et extrait par 2 fois 2 ml d'acétate d'éthyle. Un standard de $PGE_2$ est ajouté à l'extrait qui, après évaporation à sec est soumis à une chromatographie bidimensionnelle sur couche mince de silice (Merck F 254) dans les systèmes : 1) benzène, dioxane, acide acétique (60:30:3); 2) chloroforme, méthanol, acide acétique eau (90/8/1/0,8). Le standard de $PGE_2$ est révélé par l'acide phosphomolybdique à chaud. La radioactivité correspondant aux métabolites de l'acide arachidonique est mesurée grâce à un scanner Berthold. Les résultats rapportés sur la fig. 2 montrent que le composé A selon l'invention, comme l'imidazole, inhibe la formation du thromboxane $A_2$ mesuré par son produit de dégradation stable, le thromboxane. Parallèlement à cette inhibition, des quantités croissantes de $PGE_2$ sont formées. Cette observation indique que les agents pharmacoligiques testés n'inhibent pas parallèlement la cyclooxygénase.

On donnera ci-après, dans le tableau II, les résultats de la détermination des concentrations (en mole) inhibitrices à 50% ($CI_{50}$).

TABLEAU II

| Composé | $CI_{50}$ (M) |
|---|---|
| A | $2,5 \times 10^{-6}$ |
| B | $10^{-6}$ |
| C | $1,5 \times 10^{-6}$ |
| D | $1,5 \times 10^{-6}$ |
| E | $1,5 \times 10^{-6}$ |
| F | $10^{-6}$ |
| G | $1,5 \times 10^{-6}$ |
| Imidazole | $50 \times 10^{-6}$ |

La comparaison des $CI_{50}$ montre que les composés de formule I sont beaucoup plus actifs que l'imidazole dans ces conditions expérimentales.

1.2. Utilisation des endoperoxydes plaquettaires par la prostacycline-synthétase des microsomes vasculaires

Une aorte d'un lapin mâle pesant 2,5 kg est finement coupée, boryée en tampon Tris-HCl (50 mM, pH 7.5), puis homogénéisée à 0—4°C. L'homogénat est centrifugé à 10000 g × 10 minutes. Le culot de microsomes est suspendu et homogénéisé dans du Tris-HCl (50 mM) à raison de 1,5 mg protéines/ml (les protéines sont déterminées par la méthode de Lowry et al.). Une suspension de plaquettes humaines est préparée et incubée comme précédemment en présence ou en absence de microsomes aortiques (100 $\mu$g protéines/incubat) et de produit testé ($10^{-5}$M). A la fin de la réaction, l'incubat est centrifugé, le surnageant est extrait après acidification et chromatographié sur couche mince de silice dans la phase organique du système : acétate d'éthyle, 2,2,4-triméthylpentane, acide acétique, eau (110:50:20:100) en présence d'un standard de 6-ceto $PgF_{1\alpha}$.

Les radiochromatogrammes correspondant aux différents incubats sont représentés dans les fig. 3 à 6.

1) *Suspension plaquettaire* (fig. 3): 60% de la radioactivité extraite présentent un Rf = 0,25—0,27 correspondant au thromboxane $B_2$ ($TXB_2$) produit de dégradation du thromboxane $A_2$. D'autres métabolites, un produit polaire non identifié, la $PGD_2$ et un produit de Rf = 0,47 sont également séparés.

2) *Microsomes d'aorte* (fig. 4): dans les conditions expérimentales décrites, l'acide arachidonique (AA) n'est pas métabolisé. Ces résultats sont en accord avec ceux rapportés par Moncada et al. (Nature, *263*, 663—665 (1976).

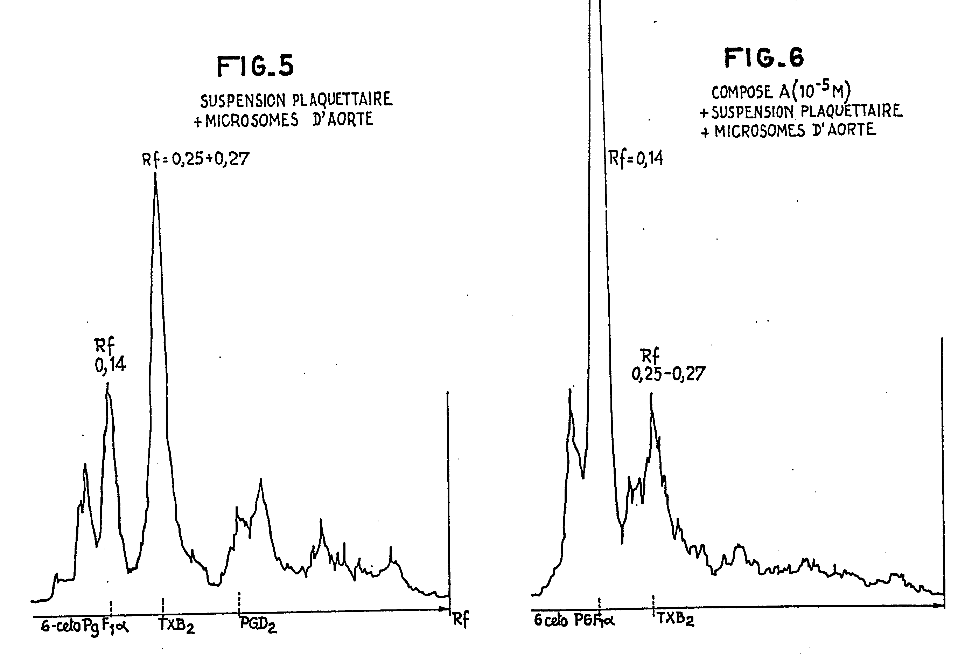

3) *Suspension plaquettaire* + *microsomes* (fig. 5): lorsque des plaquettes sont incubées en présence de microsomes d'aorte, le profil métabolique est qualitativement modifié. Un pic de radioactivité apparaît au Rf = 0,14 correspondant au 6-ceto $PgF_{1\alpha}$, produit de dégradation de la $PGI_2$. Ce pic ne représente toutefois que 18% de la radioactivité extraite et apparaît au détriment du thromboxane $B_2$ ($TXB_2$) essentiellement.

4) *Composé A* + *suspension plaquettaire* + microsomes (fig. 6): lorsque l'incubation précédente est effectuée en présence du composé A (0,01 mM), la plus grande partie (65%) de l'acide arachidonique est métabolisée en 6 ceto $PgF_{1\alpha}$, alors que la radiocactivité correspondant au thromboxane $B_2$ n'en représente que 20%. On observe parallèlement une quasi-suppression des produits les moins polaires.

Ces résultats montrent, d'une part, que le composé A inhibe fortement la synthèse du thromboxane $A_2$, qui est un agrégant, et, d'autre part, qu'il favorise dans ce système la synthèse de prostacycline $PGI_2$ qui est elle-même un anti-agrégant.

2 — Pharmacologie fonctionnelle

2.1. Agrégation plaquettaire ex vivo chez le lapin

Des groupes de trois lapins mâles sont traités par voie sous-cutanée deux jours consécutifs et leur plasma est prélevé deux heures après la seconde administration. L'agrégation plaquettaire induite à l'acide arachidonique (2 mM) est mesurée par la méthode turbidimétrique de Born.

L'analyse de courbes d'agrégation rapportée dans le tableau III montre que le composé A selon l'invention à la dose de 5 mg/kg diminue de façon très sensible l'agrégation plaquettaire induite par l'acide arachidonique chez le lapin.

TABLEAU III

Agrégation plaquettaire ex vivo chez le lapin

| Traitement | Vitesse d'agrégation (*) | Maximum (*) | Vitesse de désagrégation (*) |
|---|---|---|---|
| Témoins | 56 | 40 | 5 |
| Composé A ($5 mg \times kg^{-1}$) | 32 | 28 | 3 |

(*) moyennes pour des groupes de trois animaux

2.2 Temps de saignement à l'oreille chez le lapin

Des groupes de trois lapins mâles sont traités par voie sous-cutanée par les produits à tester. Leur temps de saignement (TS) est déterminé juste avant, puis deux heures et quatre heures après leur administration selon un protocole standardisé par un expérimentateur agissant "en aveugle".

Les temps de saignement mesurés sont rapportés dans le tableau IV. Les animaux traités par une dose unique de composé A selon l'invention (5 mg/kg) présentent un doublement de leur TS au temps deux heures après traitement: cet allongement est hautement significatif et se poursuit au temps quatre heures.

TABLEAU IV

Temps de saignement à l'oreille chez le lapin

| Traitement | Temps de saignement (secondes) * | | |
|---|---|---|---|
| | Temps 0 | Temps 2 h. | Temps 4 h. |
| Témoins | 70 ± 23 | 85 ± 31 | 87 ± 29 |
| Composé A ($5 mg \times kg^{-1}$) | 80 ± 27 | 165 ± 38 | 102 ± 19 |

* moyenne ± écart type pour des groupes de trois animaux

3 — Inhibition de l'agrégation plaquettaire induite par le collagène et mesure de l'inhibition de la synthèse de thromboxane

Du sang veineux prélevé chez des volontaires est collecté sur citrate de sodium. Le plasma riche en plaquettes (PRP) est préparé par centrifugation à 150 g x 10 min.

300 $\mu$l de PRP sont incubés en tubes siliconés dans un agrégomètre en présence ou en absence de produit à tester en solution dans 100 $\mu$l de tampon, puis 20 $\mu$g de collagène (Stago) sont ajoutés. L'agrégation plaquettaire est enregistrée par la diminution de la densité optique. Après 4 minutes, une solution d'acide acétylsalicylique est ajoutée ($2 \times 10^{-4}$M final) pour bloquer la synthèse des prostanoïdes et le thromboxane $B_2$ est dosé dans la phase liquide de l'incubat par un dosage radio-immunologique.

Les résultats sont donnés dans le tableau V.

TABLEAU V

| Incubat | Agrégation (%) | T x $B_2$ formé (pmole /ml PRP) |
|---|---|---|
| Témoin | 100 | 900 |
| Composé B ($10^{-6}$M) | 50 | 400 |
| Composé B ($10^{-5}$M) | 7 | 100 |

4 — Toxicité aigüe

Les composés de formule I ont une toxicité qui n'apparaît qu'à des doses très supérieures aux doses pharmacologiquement actives. On donnera ci-après dans le tableau VI les résultats de mesure de la $DL_{50}$ du composé A par voie intrapéritonéale chez le rat Wistar et la souris Swiss.

TABLEAU VI

$DL_{50}$ du composé A par voie intrapéritonéale chez le rat et la souris

| Espèce | Sexe | $DL_{50}$* (mg × $kg^{-1}$) |
|---|---|---|
| Rat | M | 270 ± 12 |
| ,, | F | 250 ± 16 |
| Souris | M | 166 ± 7 |
| ,, | F | 164 ± 6 |

* moyenne ± ic à 5 %

Les compositions thérapeutiques selon l'invention peuvent être utilisées notamment pour le traitement et la prévention des thromboses, en particulier des thromboses chez les diabétiques et des thromboses rétiniennes, ainsi que pour le traitement de la rétinopathie diabétique.

Ces compositions thérapeutiques selon l'invention sont administrables à l'homme par voie orale (sous forme de comprimés ou gélules) ou par voie parentérale (sous forme de solutions aqueuses, notamment pour perfusion intraveineuse).

Sous forme de doses unitaires pour l'administration par voie orale, elles peuvent contenir de 20 à 100 mg de principe actif.

La posologie journalière peut varier de 80 à 400 mg de principe actif.

**Revendications pour les Etats contractants: BE CH DE FR GB IT LI LU NL SE**

1. Composition thérapeutique ayant une activité inhibitrice de la thromboxane synthétase, caractérisée en ce qu'elle contient à titre de principe actif le (E) (hydroxy-3 octényl-1)-1 imidazole ou l'un de ses sels d'addition avec des acides pharmaceutiquement acceptables.

2. Composition thérapeutique ayant une activité antiagrégante plaquettaire, caractérisée en ce qu'elle contient à titre de principe actif le (E) (hydroxy-3 octényl-1)-1 imidazole ou l'un de ses sels d'addition avec des acides pharmaceutiquement acceptables.

3. Composition thérapeutique ayant une activité antithrombotique, caractérisée en ce qu'elle contient à titre de principe actif le (E) (hydroxy-3 octényl-1)-1 imidazole ou l'un de ses sels d'addition avec des acides pharmaceutiquement acceptables.

4. Composition thérapeutique, caractérisée en ce qu'elle contient à titre de principe actif un composé de formule

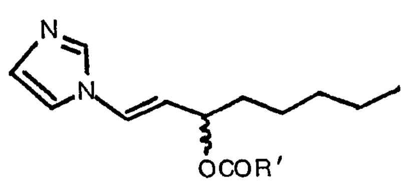

(II)

dans laquelle R' représente un radical alkyle en $C_1$ à $C_8$, un radical aryle, aryl(alkyle en $C_1$—$C_5$) ou (alkyle en $C_1$—$C_5$) aryle, le terme aryle désignant un radical hydrocarboné aromatique ayant de 6 à 10 atomes de carbone ou un radical hétéroaromatique contenant un ou deux hétéroatomes choisis parmi l'azote, l'oxygène et le soufre et ayant de 5 à 11 chaînons, ou l'un de ses sels d'addition avec des acides pharmaceutiquement acceptables.

5. Composition selon la revendication 4, caractérisée en ce qu'elle contient à titre de principe actif l'ester nicotinique du (E)-(hydroxy-3-octèn-1-yl)-1-imidazole, ou l'un de ses sels d'addition avec des acides pharmaceutiquement acceptables.

6. Composition thérapeutique, caractérisée en ce qu'elle contient à titre de principe actif un composé de formule

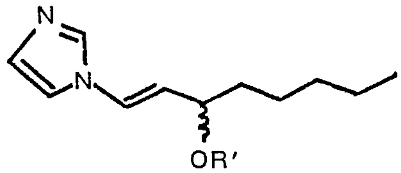

(III)

dans laquelle R′ représente un radical alkyle en $C_1$ à $C_8$, un radical aryle, aryl(alkyle en $C_1$—$C_5$) ou (alkyle en $C_1$—$C_5$) aryle, le terme aryle désignant un radical hydrocarboné aromatique ayant de 6 à 10 atomes de carbone ou un radical hétéroaromatique contenant un ou deux hétéroatomes choisis parmi l'azote, l'oxygène et le soufre et ayant de 5 à 11 chaînons, ou l'un de ses sels d'addition avec des acides pharmaceutiquement acceptables.

7. Composés de formule

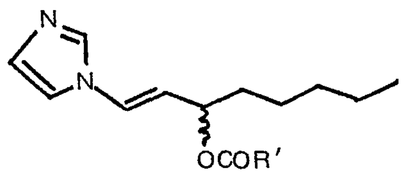

(II)

dans laquelle R′ représente un radical alkyle en $C_1$ à $C_8$, un radical aryle, aryl(alkyle en $C_1$—$C_5$) ou (alkyle en $C_1$—$C_5$) aryle, le terme aryle désignant un radical hydrocarboné aromatique ayant de 6 à 10 atomes de carbone ou un radical hétéroaromatique contenant un ou deux hétéroatomes choisis parmi l'azote, l'oxygène et le soufre et ayant de 5 à 11 chaînons, et leurs sels d'addition avec des acides pharmaceutiquement acceptables.

8. L'ester nicotinique du (E)-(hydroxy-3-octèn-1-yl)-1-imidazole, et ses sels d'addition avec des acides pharmaceutiquement acceptables.

9. Composés de formule

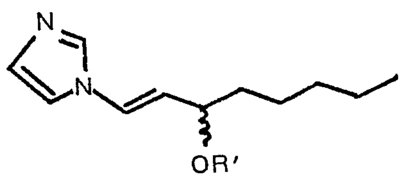

(III)

dans laquelle R′ représente un radical alkyle en $C_1$ à $C_8$, un radical aryle, aryl(alkyle en $C_1$—$C_5$) ou (alkyle en $C_1$—$C_5$) aryle, le terme aryle désignant un radical hydrocarboné aromatique ayant de 6 à 10 atomes de carbone ou un radical hétéroaromatique contenant un ou deux hétéroatomes choisis parmi l'azote, l'oxygène et le soufre et ayant de 5 à 11 chaînons, et leurs sels d'addition avec des acides pharmaceutiquement acceptables.

10. Procédé de préparation de composés de formule II selon la revendication 7, caractérisé en ce que l'on fait réagir sur un alcool de formule

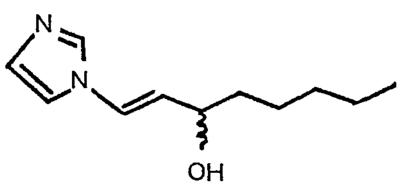

un halogénure d'acide de formule R′COX dans laquelle R′ a la signification donnée ci-dessus et X désigne un atome d'halogène, et l'on convertit éventuellement le composé obtenu en un sel par action d'un acide pharmaceutiquement acceptable.

11. Procédé de préparation de composés de formule III selon la revendication 9, caractérisé en ce que l'on fait réagir sur un alcool de formule

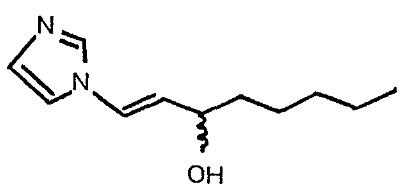

un dérivé halogéné de formule R'X dans laquelle R' a la signification donnée ci-dessus et X désigne un atome d'halogène, et l'on convertit éventuellement le composé obtenu en un sel par action d'un acide pharmaceutiquement acceptable.

**Revendications pour l'Etat contractant: AT**

1. Procédé de préparation de composés de formule

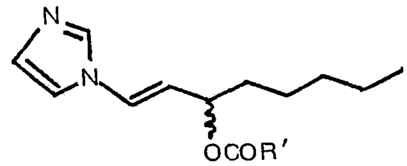

dans laquelle R' représente un radical alkyle en $C_1$ à $C_8$, un radical aryle, aryl(alkyle en $C_1$—$C_5$) ou (alkyl en $C_1$—$C_5$) aryle, le terme aryle désignant un radical hydrocarboné aromatique ayant de 6 à 10 atomes de carbone ou un radical hétéroaromatique contenant un ou deux hétéroatomes choisis parmi l'azote, l'oxygène et le soufre et ayant de 5 à 11 chaînons, et de leurs sels d'addition avec des acides pharmaceutiquement acceptables, caractérisé en ce que l'on fait réagir sur un alcool de formule

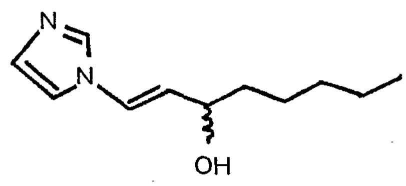

un halogénure d'acide de formule R'COX dans laquelle R' a la signification donnée ci-dessus et X désigne un atome d'halogène, et l'on convertit éventuellement le composé obtenu en un sel par action d'un acide pharmaceutiquement acceptable.

2. Procédé de préparation de composés de formule

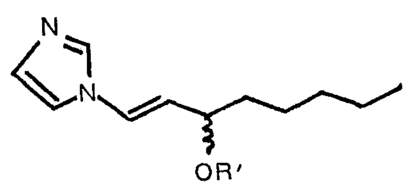

(III)

dans laquelle R' représente un radical alkyle en $C_1$ à $C_8$, un radical aryle, aryl(alkyle en $C_1$—$C_5$) ou (alkyl en $C_1$—$C_5$) aryle, le terme aryle désignant un radical hydrocarboné aromatique ayant de 6 à 10 atomes de carbone ou un radical hétéroaromatique contenant un ou deux hétéroatomes choisis parmi l'azote, l'oxygène et le soufre et ayant de 5 à 11 chaînons, et de leurs sels d'addition avec des acides pharmaceutiquement acceptables, caractérisé en ce que l'on fait réagir sur un alcool de formule

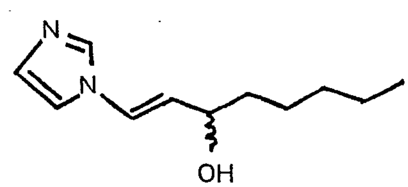

un dérivé halogéné de formule R'X dans laquelle R' a la signification donnée ci-dessus et X désigne un atome d'halogène, et l'on convertit éventuellement le composé obtenu en un sel par action d'un acide pharmaceutiquement acceptable.

**Claims for the Contracting States: BE CH DE FR GB IT LI LU NL SE**

1. Therapeutic composition having an inhibitory activity on thromboxane synthetase, characterized in that it a contains as active ingredient (E)-1-(3-hydroxy-octen-1-yl) imidazole or a pharmaceutically acceptable acid addition salt thereof.

2. Therapeutic composition having an antibloodplatelet aggregation activity, characterized in that

it a contains as active ingredient (E)-1-(3-hydroxy-octen-1-yl) imidazole or a pharmaceutically acceptable acid addition salt thereof.

3. Therapeutic composition having an antithrombotic activity, characterized in that it a contains as active ingredient (E)-1-(3-hydroxy-octen-1-yl) imidazole or a pharmaceutically acceptable acid addition salt thereof.

4. Therapeutic composition, characterized in that it contains as active ingredient a compound of the formula

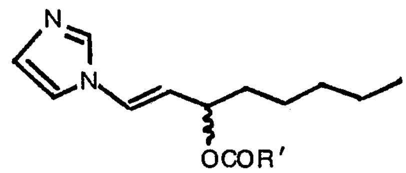

(II)

in which R′ represents a $C_{1-8}$ alkyl radical, an aryl radical, an aryl ($C_{1-5}$ alkyl) or ($C_{1-5}$ alkyl) aryl radical, the term "aryl" designating an aromatic hydrocarbon radical having 6—10 carbon atom or a 5—11 membered heteroaromatic radical containing one or two heteroatoms selected from nitrogen, oxygen and sulfur or a pharmaceutically acceptable addition salt thereof.

5. Composition as claimed in claim 4, characterized in that the it contains as active ingredient (E)-1-(3-hydroxy-octen-1-yl) imidazole nicotinic ester or a pharmaceutically acceptable acid addition salt thereof.

6. Therapeutic composition, characterized in that it contains as active ingredient a compound of the formula III

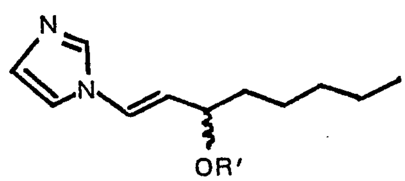

(III)

in which R′ represents a $C_{1-8}$ alkyl radical, an aryl radical, an aryl ($C_{1-5}$ alkyl) or ($C_{1-5}$) alkyl) aryl radical, the term "aryl" designating an aromatic hydrocarbon radical containing 6—10 carbon atoms or a 5—11 membered heteroaromatic radical containing one or two heteroatoms selected from nitrogen, oxygen and sulfur atoms, or a pharmaceutically acceptable acid addition salt thereof.

7. Compounds having the formula (II)

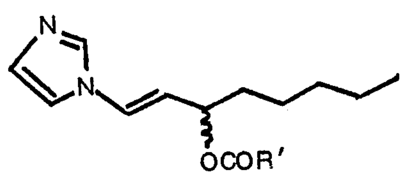

(II)

in which R′ represents a $C_{1-8}$ alkyl radical, an aryl radical, an aryl ($C_{1-5}$ alkyl) or ($C_{1-5}$ alkyl) aryl radical, the term "aryl" designating an aromatic hydrocarbon radical having 6—10 carbon atoms or a 5—11 membered heteroaromatic radical containing one or two heteroatoms selected from nitrogen, oxygen and sulfur atoms, and the pharmaceutically acceptable acid addition salts thereof.

8. (E)-1-(3-hydroxy-octen-1-yl) imidazole nicotinic ester and its pharmaceutically acceptable acid addition salts.

9. Compounds having the formula III

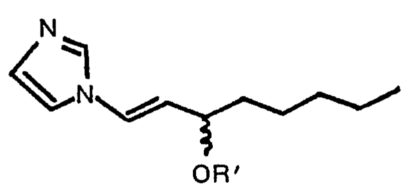

(III)

in which R′ represents a $C_{1-8}$ alkyl radical, an aryl radical, an aryl ($C_{1-5}$ alkyl) or ($C_{1-5}$ alkyl) aryl radical, the term "aryl" designating an aromatic hydrocarbon radical having 6—10 carbon atoms or a 5—11 membered heteroaromatic radical containing one or two heteroatoms selected from nitrogen, oxygen and sulfur atoms, and the pharmaceutically acceptable acid addition salts thereof.

10. Process for the preparation of compounds of the formula II as claimed in claim 7, characterized in that an alcohol of the formula

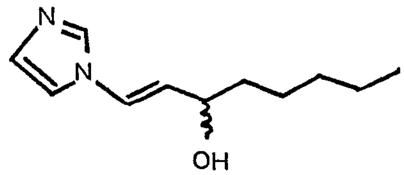

is reacted with an acid halide of the formula R'—COX in which R' has the above-defined meaning and X is a halogen atom, and optionally the obtained compound is converted into a salt by reaction with a pharmaceutically acceptable acid.

11. Process for the preparation of compounds of the formula III as claimed in claim 9, characterized in that an alcohol of the formula

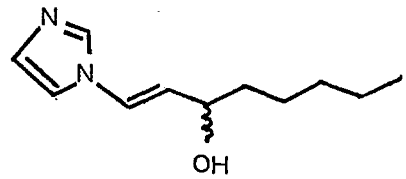

is reacted with a halogenated derivative of the formula R'X in which R' has the above-defined meaning and X is a halogen atom, and optionally the obtained compound is converted into a salt by reaction with a pharmaceutically acceptable acid.

**Claims for the Contracting State: AT**

1. Process for the preparation of compounds having the formula (II)

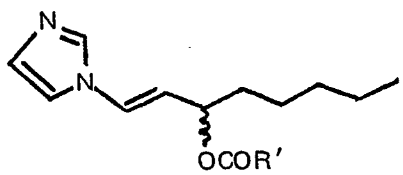

(II)

in which R' represents a $C_{1-8}$ alkyl radical, an aryl radical, an aryl $(C_{1-5})$ alkyl or ($C_{1-5}$ alkyl) aryl radical, the term "aryl" designating an aromatic hydrocarbon radical having 6—10 carbon atoms or a 5—11 membered heteroaromatic radical containing one or two heteroatoms selected from nitrogen, oxygen and sulfur atoms, and of their pharmaceutically acceptable acid addition salts, characterized in that an alcohol of the formula

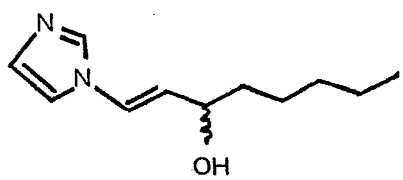

is reacted with an acid halide of the formula R'—COX in which R' has the above-defined meaning and X is a halogen atom, and optionally the obtained compound is converted into a salt by reaction with a pharmaceutically acceptable acid.

2. Process for the preparation of compounds having the formula (III)

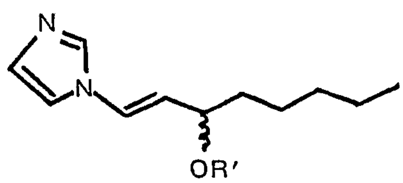

(III)

in which R' represents a $C_{1-8}$ alkyl radical, an aryl radical, an aryl ($C_{1-5}$ alkyl) or ($C_{1-5}$ alkyl) aryl radical, the term "aryl" designating an aromatic hydrocarbon radical having 6—10 carbon atoms or a 5—11 membered heteroaromatic radical containing one or two heteroatoms selected from nitrogen, oxygen

and sulfur atoms, and of their pharmaceutically acceptable acid addition salts, characterized in that an alcohol of the formula

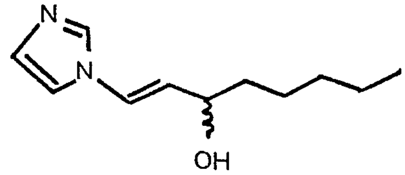

is reacted with a halogenated derivative of the formula R'X in which R' has the above-defined meaning and X is a halogen atom, and optionally the obtained compound is converted into a salt by reaction with a pharmaceutically acceptable acid.

**Patentansprüche für die Vertragsstaaten: BE CH DE FR GB IT LI LU NL SE**

1. Therapeutische Zubereitung mit hemmender Wirkung auf die Thromboxan-Synthetase, dadurch gekennzeichnet, daß sie als Wirkstoff das (E)-1-(3-Hydroxy-1-octenyl)-imidazol oder eines seiner Additionssalze mit pharmazeutisch unbedenklichen Säuren enthält.

2. Therapeutische Zubereitung mit Wirkung gegen die Blutkörperchenzusammenballung, dadurch gekennzeichnet, daß sie als Wirkstoff das (E)-1-(3-Hydroxy-1-octenyl)-imidazol oder eines seiner Additionssalze mit pharmazeutisch unbedenklichen Säuren enthält.

3. Therapeutische Zubereitung mit antithrombotischer Aktivität, dadurch gekennzeichnet, daß sie als Wirkstoff das (E)-1-(3-Hydroxy-1-octenyl)-imidazol oder eines seiner Additionssalze mit pharmazeutisch unbedenklichen Säuren enthält.

4. Therapeutische Zubereitung, dadurch gekennzeichnet, daß sie als Wirkstoff eine Verbindung der Formel

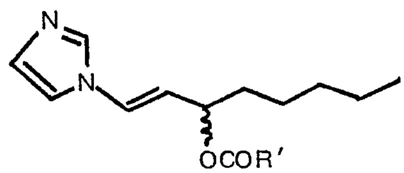

(II)

in der R' eine $C_1$—$C_8$-Alkylrest, ein Arylrest, ein Arylalkylrest mit 1 bis 5 C-Atomen in der Alkylgruppe oder ein Alkylarylrest mit 1 bis 5 C-Atomen in der Alkylgruppe ist, wobei der Ausdruck Aryl einen aromatischen Kohlenwasserstoffrest mit 6 bis 10 Kohlenstoffatomen oder einen heteroaromatischen Rest bedeutet, der eine oder zwei aus Stickstoff, Sauerstoff und Schwefel ausgewählte Heteroatome enthält und 5 bis 11 Kettenglieder hat, oder eines seiner Additionssalze mit pharmazeutisch unbedenklichen Säuren enthält.

5. Zubereitung nach Anspruch 4, dadurch gekennzeichnet, daß sie als Wirkstoff den Nicotinsäureester des (E)-1-(3-Hydroxy-1-octenyl)-imidazols oder eines seiner Additionssalze mit pharmazeutisch unbedenklichen Säuren enthält.

6. Therapeutische Zubereitung, dadurch gekennzeichnet, daß sie als Wirkstoff eine Verbindung der Formel

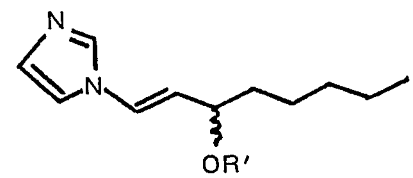

(III)

in der R' ein $C_1$—$C_8$-Alkylrest, ein Arylrest, ein Arylalkylrest mit 1 bis 5 C-Atomen im Alkylrest oder ein Alkylarylrest mit 1 bis 5 C-Atomen im Alkylrest ist, wobei der Ausdruck Aryl einen aromatischen Kohlenwasserstoffrest mit 6 bis 10 Kohlenstoffatomen oder einen heteroaromatischen Rest bedeutet, der ein oder zwei aus Stickstoff, Sauerstoff und Schwefel ausgewählt Heteroatome enthält und 5 bis 11 Kettenglieder hat, oder eines seiner Additionssalze mit pharmazeutisch unbedenklichen Säuren enthält.

7. Verbindungen der Formel

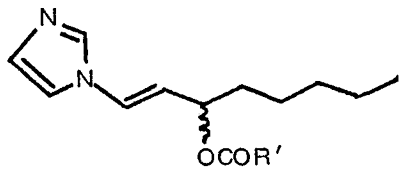

(II)

in der R' einen $C_1$—$C_8$-Alkylrest, einen Arylrest, einen Arylalkylrest mit 1 bis 5 C-Atomen im Alkylrest oder einen Alkylarylrest mit 1 bis 5 C-Atomen im Alkylrest darstellt, wobei der Ausdruck Aryl einen aromatischen Kohlenwasserstoffrest mit 6 bis 10 Kohlenstoffatomen oder einen heteroaromatischen Rest bedeutet, der ein oder zwei aus Stickstoff, Sauerstoff und Schwefel ausgewählte Heteroatome enthält und 5 bis 11 Kettenglieder hat, und ihre Additionssalze mit pharmazeutisch unbedenklichen Säuren.

8. Nicotinsäureester von (E)-1-(3-Hydroxy-1-octenyl)-imidazol und seine Additionssalze mit pharmazeutisch unbedenklichen Säuren.

9. Verbindungen der Formel

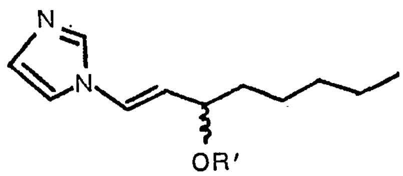

(III)

in der R' einen $C_1$—$C_8$-Alkylrest einen Arylrest, einen Arylalkylrest mit 1—5 C-Atomen in Alkylrest oder einen Alkylarylrest mit 1—5 C-Atomen im Alkylrest darstellt, wobei der Ausdruck Aryl einen aromatischen Kohlenwasserstoffrest mit 6 bis 10 Kohlenstoffatomen oder einen heteroaromatischen Rest bedeutet, der ein oder zwei aus Stickstoff, Sauerstoff und Schwefel ausgewählt Heteroatome enthält und 5 bis 11 Kettenglieder hat, und ihre Additionssalze mit pharmazeutische unbedenklichen Säuren.

10. Verfahren zur Herstellung von Verbindungen der Formel II gemäß Anspruch 7, dadurch gekennzeichnet, daß man mit einem Alkohol der Formel

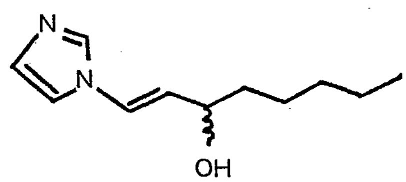

ein Säurehalogenid der Formel R'COX, in der R' die vorstehend angegebene Bedeutung hat und X eine Halogenatom bezeichnet, umsetzt und die erhaltene Verbindung gegebenenfalls durch Einwirkung einer pharmazeutisch unbedenklichen Säure in ein Salz umwandelt.

11. Verfahren zur Herstellung von Verbindungen der Formel III gemäß Anspruch 9, dadurch gekennzeichnet, daß man mit einem Alkohol der Formel

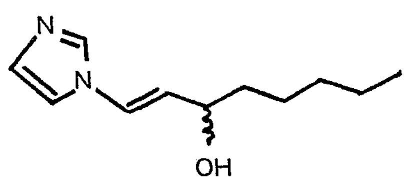

ein halogeniertes Derivat der Formel R'X, in der R' die vorstehend angegebene Bedeutung hat und X ein Halogenatom bezeichnet, umsetzt und die erhaltene Verbindung gegebenenfalls durch Einwirkung einer pharmazeutisch unbedenklichen Säure in ein Salz umwandelt.

**Patentansprüche für den Vertragsstaat: AT**

1. Verfahren zur Herstellung von Verbindungen der Formel

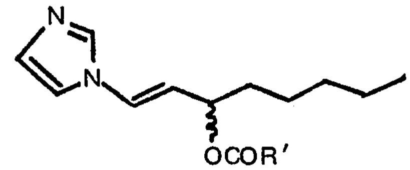

in der R' ein $C_1$—$C_8$-Alkylrest, ein Arylrest, einArylalkylrest mit 1—5 C-Atomen im Alkylrest oder ein Alkylarylrest mit 1—5 C-Atomen in Alkylrest ist, wobei der Ausdruck Aryl einen aromatischen Kohlenwasserstoffrest mit 6 bis 10 Kohlenstoffatomen oder einen heteroaromatischen Rest bedeutet, der ein oder zwei aus Stickstoff, Sauerstoff und Schwefel ausgewählte Heteroatome enthält und 5 bis 11 Kettenglieder hat, und ihren Additionssalzen mit pharmazeutisch unbedenklichen Säuren, dadurch gekennzeichnet, daß man mit einem Alkohol der Formel

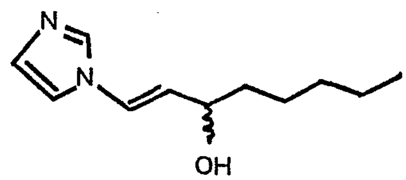

ein Säurehalogenid der Formel R'COX, in der R' die vorstehend angegebene Bedeutung hat und X ein Halogenatom bezeichnet, umsetzt und die erhaltene Verbindung gegebenenfalls durch Einwirkung einer pharmazeutisch unbedenklichen Säure in ein Salz umwandelt.

2. Verfahren zur Herstellung von Verbindungen der Formel

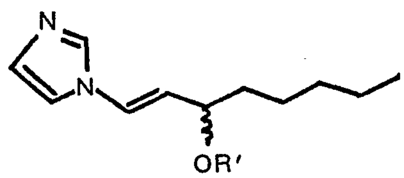

in der R' einen $C_1$—$C_8$-Alkylrest, einen Arylrest, einen Arylalkylrest mit 1—5 C-Atomen im Alkylrest oder einen Alkylarylrest mit 1—5 C-Atomen im Alkylrest bezeichnet, wobei der Ausdruck Aryl einen aromatischen Kohlenwasserstoffrest mit 6 bis 10 Kohlenstoffatomen oder einen heteroaromatischen Rest bezeichnet, der ein oder zwei aus Stickstoff, Sauerstoff und Schwefel ausgewählte Heteroatome enthält und 5 bis 11 Kettenglieder hat, und ihren Additionssalzen mit pharmazeutisch unbedenklichen Säuren, dadurch gekennzeichnet, daß man mit einem Alkohol der Formel

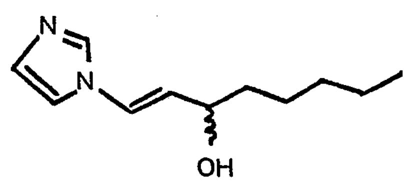

ein halogeniertes Derivat der Formel R'X, in der R' die vorstehend angegebene Bedeutung hat und X ein Halogenatom bezeichnet, umsetzt und die erhaltene Verbindung gegebenenfalls durch Einwirkung einer pharmazeutisch unbedenklichen Säure in ein Salz umwandelt.

FIG_1

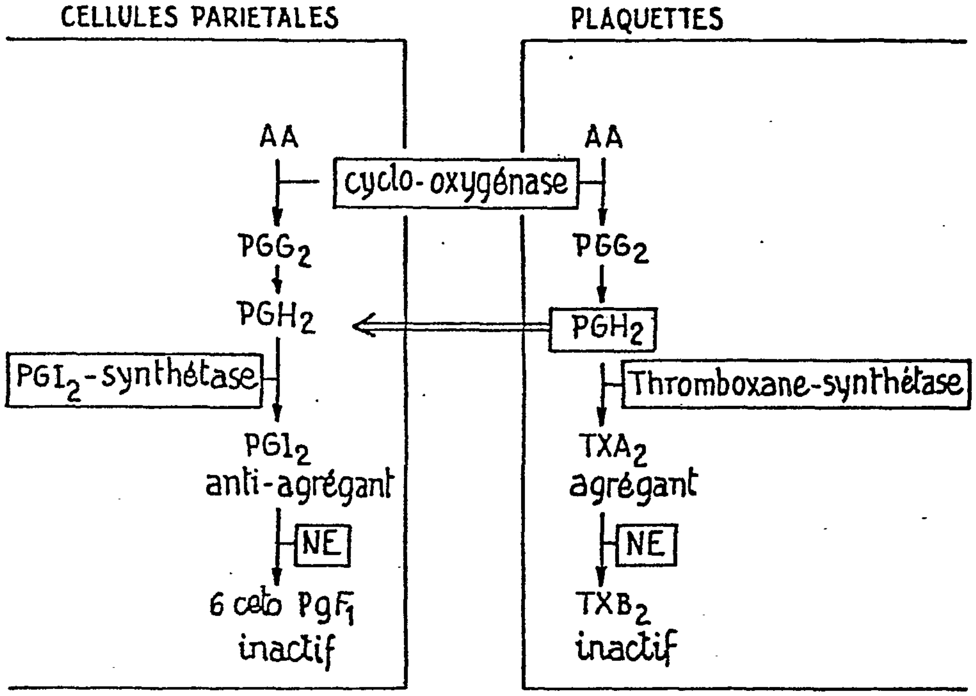


NE : dégradation non enzymatique

FIG_2 INHIBITION DE LA THROMBOXANE SYNTHETASE DES PLAQUETTES HUMAINES

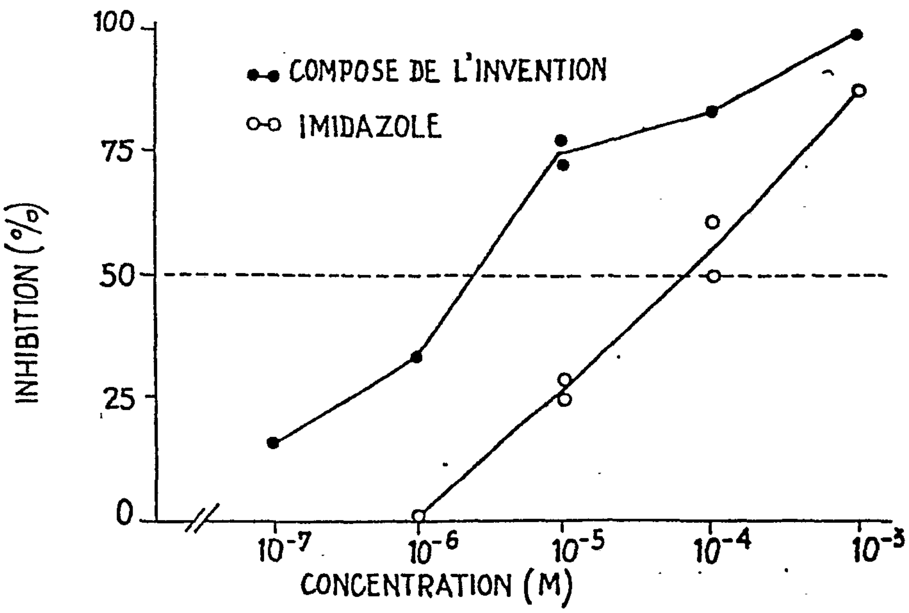

FIG.4

MICROSOMES D'AORTE

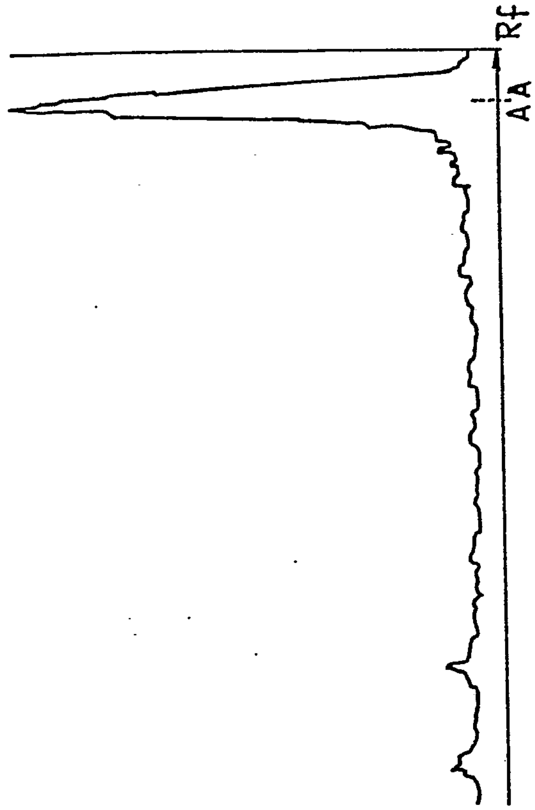


FIG.3

SUSPENSION PLAQUETTAIRE

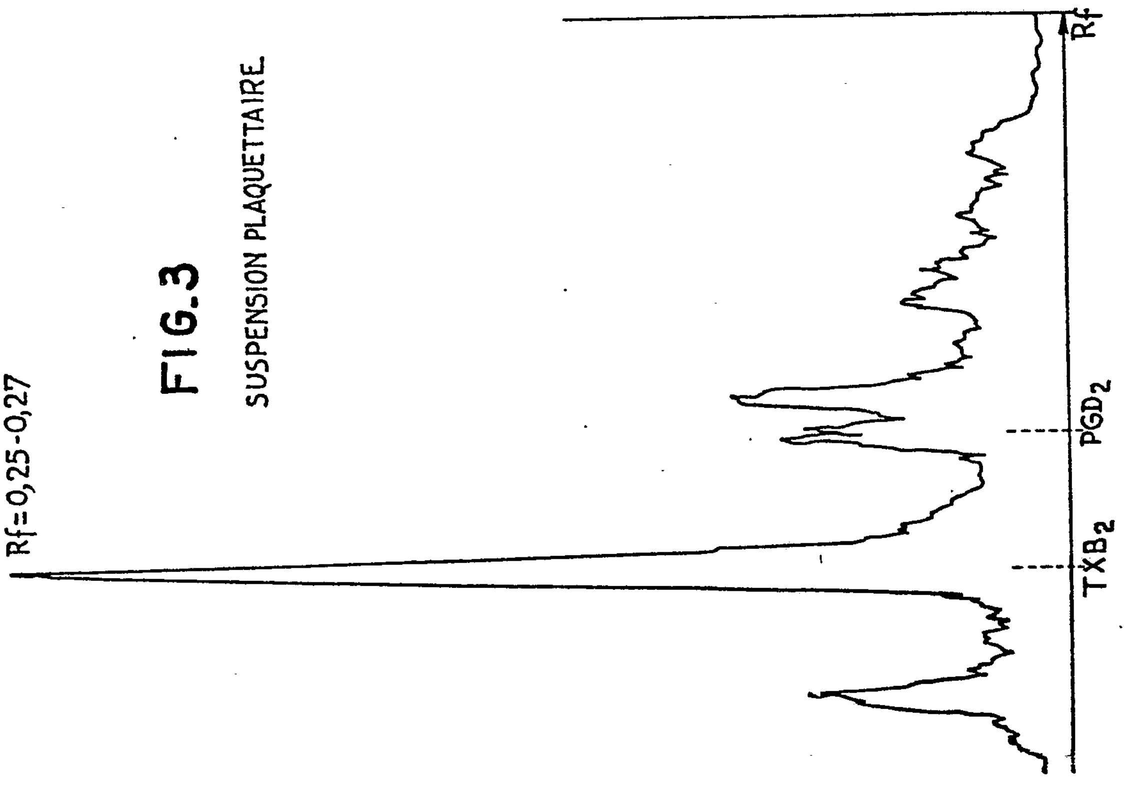

FIG. 6

COMPOSE A (10$^{-5}$M)
+ SUSPENSION PLAQUETTAIRE
+ MICROSOMES D'AORTE

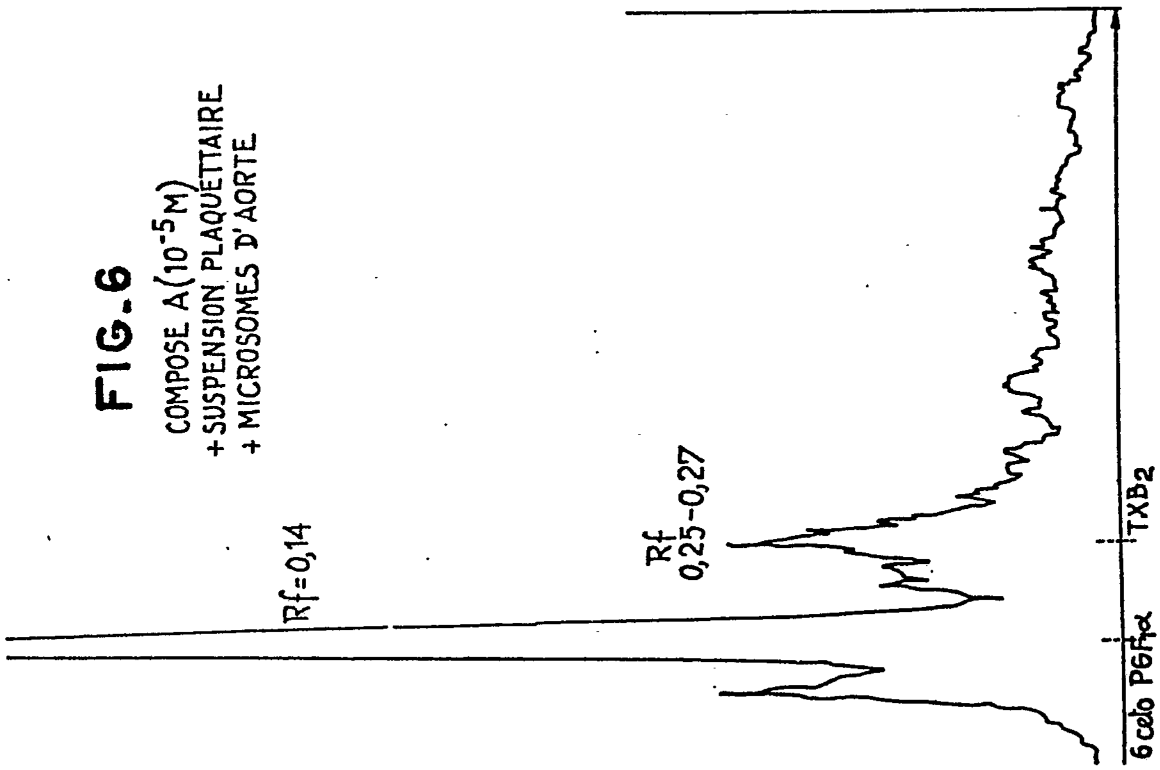


FIG. 5

SUSPENSION PLAQUETTAIRE
+ MICROSOMES D'AORTE